# DEMANDE DE BREVET EUROPEEN

(11) **EP 2 113 240 A1**
(43) Date de publication de la demande: **04.11.2009**
(21) Numéro de dépôt: 09158868.1
(22) Date de dépôt: 27.04.2009
(51) Int. Cl.: A61K 8/06, A61K 8/31, A61K 8/41, A61K 8/86, A61K 8/92, A61Q 5/00, A61Q 5/02, A61Q 5/12

(54) **Composition cosmétique comprenant une émulsion obtenue par procédé PIT.**

(30) Priorité: 28.04.2008 FR 0852850
(71) Demandeur: L'Oréal, 75008 Paris (FR)
(72) Inventeur: Mathonneau, Estelle, 75010, Paris (FR)
(74) Mandataire: Dossmann, Gérard

(57) **Abrégé**

La présente invention se rapporte à une composition cosmétique aqueuse pour le traitement des matières kératiniques, telles que les cheveux, comprenant une émulsion directe (A1) obtenue par un procédé PIT et un ou plusieurs polymères cationiques, caractérisée par le fait que l'émulsion directe (A1) comporte :
a) une phase lipophile comprenant une ou plusieurs huile(s) minérale(s) et une ou plusieurs huile(s) végétale(s),
b) un système émulsionnant comprenant un ou plusieurs tensioactif(s) non ionique(s) oxyéthyléné(s) et/ou glycérolé(s) de HLB allant de 8 à 18, et
c) une phase aqueuse.

Un autre objet de l'invention porte sur l'utilisation de cette composition pour laver et/ou conditionner les cheveux.

## Description

La présente invention se rapporte à une composition cosmétique aqueuse, sous forme d'émulsion directe, obtenue par inversion de phase, et ses utilisations dans le domaine du soin et/ou du lavage des matières kératiniques.

La fabrication d'émulsions directes concentrées en huile est souvent difficile, et les émulsions obtenues sont souvent instables. Ces émulsions sont classiquement obtenues par voie mécanique, par exemple par émulsification avec un rotor stator ou à l'aide d'un homogénéisateur haute-pression, parce qu'il faut beaucoup d'énergie mécanique pour diviser la phase dispersée en petites gouttes. Pour stabiliser ces émulsions, on y ajoute généralement des tensioactifs émulsionnants du type huile-dans-eau, c'est-à-dire de HLB (HLB = Hydrophilic Lipophilic Balance) allant de 8 à 18, émulsionnants qui, grâce à leur structure amphiphile, se placent à l'interface phase huileuse / phase aqueuse, et stabilisent ainsi les gouttelettes d'huiles dispersées. Malgré la présence d'émulsionnants, les émulsions peuvent avoir tendance à se déstabiliser (coalescence puis séparation des phases aqueuse et huileuse avec relargage d'huile). Pour améliorer la stabilité de ces émulsions, on peut augmenter la concentration en émulsionnants ; toutefois, une forte concentration en émulsionnants peut conduire à un toucher rêche, collant ou poisseux, ainsi qu'à des problèmes d'innocuité vis-à-vis de la peau, des yeux et du cuir chevelu.

Pour résoudre les problèmes de stabilité des émulsions directes classiques, il a été proposé de réaliser des émulsions directes obtenues par inversion de phase en température (émulsions PIT), dans lesquelles la taille moyenne des globules constituant la phase huileuse est comprise, de préférence entre 0,1 et 4 µm (100 à 4000 nm). Le principe d'émulsification par inversion de phase en température (en anglais : Phase Inversion Temperature ou PIT) est, dans son principe, bien connu de l'homme de l'art ; il a été décrit en 1968 par K. Shinoda (J. Chem. Soc. Jpn., 1968, 89, 435). I1 a été montré que cette technique d'émulsification permet d'obtenir des émulsions fines stables (K. Shinoda et H. Saito, J. Colloïd Interface Sci., 1969, 30, 258). Cette technologie a été appliquée en cosmétique dès 1972 par Mitsui et al. (Application of the phase-inversion- temperature method to the emulsification of cosmetics ; T. Mitsui, Y. Machida and F. Harusawa, American. Cosmet. Perfum., 1972, 87, 33).

Le principe de cette technique est le suivant : on réalise le mélange d'une phase aqueuse et d'une phase huileuse, que l'on porte à une température supérieur à la température de PIT, température d'inversion de phase du système, qui est la température à laquelle l'équilibre entre les propriétés hydrophile et lipophile du (ou des) émulsionnant(s) mis en oeuvre est atteint ; à température élevée, c'est-à-dire supérieure à la température d'inversion de phase (> T_{PIT}), l'émulsion est de type eau-dans-huile, et, au cours de son refroidissement, cette émulsion s'inverse à la température d'inversion de phase, pour devenir une émulsion de type huile-dans-eau, et ceci étant passée auparavant par un état de microémulsion. Ce procédé permet d'obtenir facilement des émulsions dont les gouttelettes de la phase huileuse ont un diamètre moyen en volume inférieur à 4 µm.

Or, il subsiste encore le besoin d'améliorer les propriétés cosmétiques (toucher, lissage, démêlage) des cheveux traités par ces compositions sous forme d'émulsions PIT.

Le but de la présente invention est d'obtenir des compositions lavantes et/ou conditionnantes, qui soient stables, et qui améliorent les propriétés cosmétiques des cheveux.

La demanderesse a trouvé de manière surprenante que le choix de composés particuliers dans des proportions particulières pour réaliser ces émulsions permettait d'améliorer l'efficacité des compositions au niveau des propriétés cosmétiques, notamment en terme de toucher.

La présente invention a donc pour objet une composition cosmétique aqueuse pour le traitement des matières kératiniques, telles que les cheveux, comprenant une émulsion directe (A1) obtenue par un procédé PIT et un ou plusieurs polymère(s) cationique(s), caractérisée par le fait que l'émulsion directe (A1) comporte :
a) une phase lipophile comprenant une ou plusieurs huile(s) minérale(s) et une ou plusieurs huile(s) végétale(s),
b) un système émulsionnant comprenant un ou plusieurs tensioactif(s) non ionique(s) oxyéthyléné(s) et/ou glycérolé(s) de HLB allant de 8 à 18, et
c) une phase aqueuse.

De préférence, le rapport pondéral de la quantité d'huiles minérale(s) et végétale(s) sur la quantité de tensioactif(s) non ionique(s) oxyéthyléné(s) et/ou glycérolé(s) de HLB allant de 8 à 18 est inférieur à 4.

Par "émulsion directe", on entend au sens de la présente invention, une émulsion à phase continue aqueuse correspondant à la dispersion d'une phase lipophile dans ladite phase aqueuse continue. On parle souvent de manière simplifiée d'émulsion H/E (huile dans l'eau).

Par "composition finale", on entend la composition résultant du mélange de l'émulsion PIT et de la composition aqueuse comprenant le ou les polymère(s) cationique(s).

Les compositions finales selon l'invention se caractérisent par :
- leur aspect qui peut aller de l'opaque au translucide, voire transparente,
- le pH qui va de 3 à 8,
- la petite taille des gouttelettes de phase lipophile,
- lorsqu'elles sont rincées, une bonne ré-émulsification de la phase lipophile lors du rinçage à l'eau pour limiter le résidu lipophile sur le cuir chevelu ou les cheveux.

En outre, ces compositions sont agréables à utiliser du fait de la phase aqueuse externe et elles allient ainsi efficacité et agrément cosmétique. Le toucher est non gras à l'application et la pénétration de la composition est rapide dans le cheveu.

Les compositions peuvent être utilisées de manière différente en fonction des applications souhaitées : elles peuvent être par exemple utilisées en tant que shampooings ou utilisées en tant que soins capillaires et en particulier d'après-shampooings, en post ou prétraitement, de façon rincée ou non rincée.

La composition selon l'invention de préférence ne comprend pas de filtres solaires. De préférence elle ne contient pas de colorants capillaires directs ou d'oxydation apportant une coloration aux fibres kératiniques.

D'autres caractéristiques, aspects, objets et avantages de la présente invention apparaîtront encore plus clairement à la lecture de la description et des exemples qui suivent.

A moins d'une indication différente, les bornes des gammes de valeurs qui sont données dans le cadre de la présente invention sont incluses dans ces gammes.

### Phase lipophile

On entend par "phase lipophile" dans la présente demande, la phase contenant les composés lipophiles que sont notamment les huiles de l'invention et éventuellement les composés lipophiles additionnels tels que les huiles autres que les huiles minérales et les huiles végétales, les gommes, les pâtes et les cires.

Les émulsionnants du système émulsionnant ne font pas partie de la phase lipophile telle que définie ci-dessus.

La phase lipophile appelée aussi phase huileuse ou grasse, comporte une ou plusieurs huile(s) minérale(s) et une ou plusieurs huile(s) végétale(s).

On entend par "huile" tout composé lipophile, non ionique, insoluble dans l'eau et liquide à température ambiante (25 °C) et sous pression atmosphérique. Par insoluble dans l'eau, on entend au sens de la présente invention un composé dont la solubilité à pH spontané dans l'eau à 25 °C et à pression atmosphérique est inférieur à 1 % et de préférence inférieure à 0,5 %. Les huiles ont de préférence une température de fusion inférieure à 5°C et une viscosité inférieure à 500 cPs à 25 °C à un taux de cisaillement de 1s⁻¹.

En particulier, on entend par "huile végétale" une huile telle que définie ci-dessus, cosmétiquement acceptable, extraite d'une espèce appartenant au règne végétal.

Les huiles végétales utilisées dans la présente invention sont choisies parmi les huiles végétales habituellement employées dans le domaine cosmétique.

A titre d'exemple d'huile végétale utilisable dans les compositions de l'invention, on peut citer :
- l'huile d'amande douce,
- l'huile d'argan,
- l'huile d'avocat,
- l'huile d'arachide,
- l'huile de camélia,
- l'huile de carthame,
- l'huile de calophyllum,
- l'huile de colza,
- l'huile de coprah,
- l'huile de coriandre,
- l'huile de courge,
- l'huile de germes de blé,
- l'huile de jojoba,
- l'huile de lin,
- l'huile de macadamia,
- l'huile de germes de maïs,
- l'huile de noisette,
- l'huile de noix,
- l'huile de vernonia,
- l'huile de noyau d'abricot,
- l'huile d'olive,
- l'huile d'onagre,
- l'huile de palme,
- l'huile de passiflore,
- l'huile de pépins de raisin,
- l'huile de rosier,
- l'huile de ricin,
- l'huile de seigle,
- l'huile de sésame,
- l'huile de son de riz,
- l'huile de soja, et
- l'huile de tournesol.

Les huiles végétales selon l'invention n'ont pas subi de transformation chimique après extraction, à l'exception d'une éventuelle hydrogénation.

Parmi les huiles végétales citées ci-dessus, on utilise de préférence l'huile d'olive, l'huile d'argan, l'huile d'avocat, l'huile de colza, l'huile de jojoba, l'huile de soja, l'huile de tournesol, et de manière plus préférée l'huile d'olive.

On entend par "huiles minérales" des hydrocarbures sous forme d'huiles, linéaires ou ramifiés, saturés ou insaturés, d'origine minérale ou synthétique, et pouvant être hydrogénées.

Les huiles minérales utilisées dans la présente invention sont choisies parmi les huiles minérales, telles que définies ci-dessus, habituellement employées dans le domaine cosmétique.

A titre d'exemples d'huiles minérales utilisables dans la présente invention, on peut citer :
- les mélanges d'huiles hydrocarbonées dérivées du pétrole (nom INCI : Mineral Oil),
- l'huile de paraffine, volatile ou non volatile,
- l'huile de vaseline,
- les polyoléfines et en particulier les polydécènes,
- les isoparaffines telles que l'isohexadécane, l'isododécane et les polyisobutylènes hydrogénés tels que l'huile de Parléam® commercialisée par la société NOF Corporation (nom INCI : Hydrogenated Polyisobutene).

Parmi les huiles minérales citées ci-dessus, on utilise de préférence :
- les mélanges d'huiles hydrocarbonées dérivées du pétrole,
- l'huile de paraffine, volatile ou non volatile, et,
- l'huile de vaseline, et
- les polyoléfines et en particulier les polydécènes.

Par "polydécènes", on entend tous composés de formule C₁₀ₙH₍₂₀ₙ₎₊₂ avec n variant de 3 à 9, répondant à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes. Parmi ces composés, on choisit plus particulièrement selon l'invention ceux pour lesquels dans la formule, n varie de 3 à 7.

On peut citer, à titre d'exemple, et de préférence, le produit vendu sous la dénomination SILKFLO® 366 NF POLYDECENE par la société AMOCO CHEMICAL, ceux vendus sous la dénomination NEXBASE® 2002 FG, 2004 FG, 2006 FG et 2008 FG par la société FORTUM.

L'huile minérale préférée est l'huile de vaseline.

Dans un mode préféré de l'invention, l'huile minérale est l'huile de vaseline et l'huile végétale est choisie parmi l'huile d'olive, l'huile d'argan, l'huile d'avocat, l'huile de colza, l'huile de jojoba, l'huile de soja, l'huile de tournesol.

De préférence, l'huile (les huiles) végétale(s) et l'huile (les huiles) minérale(s) sont présentes dans la composition dans un rapport pondéral huile(s) végétale(s)/huile(s) minérale(s) inférieur ou égal à 1.

Encore plus préférentiellement, ce rapport va de 0,1 à 0,9 et mieux encore de 0,2 à 0,7.

La quantité totale d'huiles minérale(s) et végétale(s) présentes dans la composition finale va de préférence de 0,1 à 30 % en poids, et mieux encore de 0,5 à 20 % en poids, du poids total de la composition finale.

De préférence, le rapport pondéral de la quantité d'huiles minérale(s) et végétale(s) sur la quantité de tensioactif(s) non ionique(s) oxyéthyléné(s) et/ou glycérolé(s) de HLB allant de 8 à 18 est inférieur à 4, de préférence il va de 2,5 à 3,5.

Les autres constituants lipophiles pouvant être présents dans la phase lipophile sont par exemple des huiles additionnelles différentes de celles décrites ci-dessus à savoir :
- les huiles hydrocarbonées d'origine animale, telles que le persqualène (ou squalane) ;
- les esters et les éthers de synthèse, notamment d'acides gras, comme les huiles de formules R¹COOR² et R¹OR² dans lesquelles R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl sarcosinate) commercialisé sous la dénomination Eldew SL 205 par la société Ajinomoto ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényldiméthicones, les diphénylméthyldiphényltrisiloxanes, les 2-phényléthyltriméthyl-siloxysilicates, et les polyméthylphénylsiloxanes ;
- les huiles fluorées telles que celles partiellement hydrocarbonées et/ou siliconées comme celles décrites dans le document JP-A-2-295912 ;
- les éthers tels que l'éther dicaprylique (nom CTFA : Dicaprylyl ether) ; et les benzoates d'alcools gras en C₁₂-C₁₅ (Finsolv TN de FINETEX) ;
- et leurs mélanges.

A titre d'huile additionnelle utilisable, on peut également citer les esters gras. Les esters gras sont de préférence ceux obtenus à partir d'un alcool à chaîne linéaire ou ramifiée, ayant de 1 à 17 atomes de carbone et d'un acide gras à chaîne linéaire ou ramifiée, ayant de 3 à 18 et de préférence de 12 à 17 atomes de carbone.

Comme esters gras utilisables, on peut citer plus particulièrement le myristate de butyle, le laurate de butyle, le stéarate de butyle, le stéarate d'isopropyle, l'isostéarate d'isotéaryle, le palmitate d'éthyl-2-hexyle (ou palmitate d'octyle), le pelargonate d'éthyl-2-hexyle (ou pelargonate d'octyle), le stéarate d'éthyl-2 hexyle (ou stéarate d'octyle), le myristate d'octyl-2-dodécyle, l'hydroxystéarate d'éthyl-2-hexyle (ou hydroxystéarate d'octyle), le laurate d'isopropyle, le myristate d'isopropyle, le palmitate d'isopropyle, le palmitate d'isobutyle, le stéarate d'isobutyle, le stéarate d'isocétyle, l'adipate de diisopropyle, l'adipate de di-éthyl-2 hexyle (ou adipate de di-octyle), l'adipate de diisocétyle, le succinate d'éthyl-2-hexyle (ou succinate d'octyle), le sébacate de diisopropyle, le malate d'éthyl-2-hexyle (ou malate d'octyle), le caprate/caprylate de pentaérythritol, le pentaérythritol de tétraisostéarate, l'hexanoate d'éthyl-2-hexyle (ou hexanoate d'octyle), le laurate d'éthyle, le myristate de méthyle, l'octanoate d'octyldodécyle, le néopentanoate d'isodécyle, le néopentanoate d'isostéaryle, l'isononanoate de cétéaryle, l'isononanoate d'isodécyle, l'isononanoate d'isononyle, l'isononanoate d'isotridécyle, le lactate de lauryle, le lactate de myristyle, le lactate de cétyle, le myristate d'éthyle, le propionate de myristyle, l'éthyl-2-hexanoate d'éthyl-2-hexyle (ou éthyl-2-hexanoate d'octyle), l'octanoate d'éthyl-2-hexyle (ou octanoate d'octyle), le caprate/caprylate d'éthyl-2-hexyle (ou caprate/caprylate d'octyle), le monococoate d'éthyl-2-hexyle (ou monococoate d'octyle), le palmitate de méthyle, le myristate d'isobutyle, le palmitate d'éthyle, le laurate d'isohexyle, le laurate d'hexyle, l'isostéarate d'isopropyle, et leurs mélanges.

A titre d'exemple d'autres constituants lipophiles pouvant être présents dans la phase lipophile, on peut citer les cires et notamment les cires d'origine végétales comme la cire de carnauba ou la cire de candelia ; les gommes telles que les gommes de silicone ; les résines de silicone ; et leurs mélanges, les céramides et en particulier la N-oléyldihydrosphingosine.

### Système émulsionnant

Le système émulsionnant utilisé dans la composition selon l'invention comprend un ou plusieurs tensioactif(s) non-ionique(s) oxyéthyléné(s) et/ou glycérolé(s) de HLB allant de 8 à 18, et de préférence de 10 à 16.

Les émulsionnants présentent la particularité de faciliter la dispersion de deux phases insolubles l'une dans l'autre. La HLB (Hydrophilic Lipophilic balance) est le rapport entre la partie hydrophile et la partie lipophile dans leur molécule. Ce terme HLB est bien connu de l'homme du métier et est décrit dans "The HLB system. A time-saving guide to Emulsifier Selection" (published by ICI Americas Inc ; 1984).

Le système émulsionnant peut comprendre, en particulier, un ou plusieurs tensioactif(s) non-ionique(s) oxyéthyléné(s) et/ou glycérolé(s) choisi(s) parmi les alcools gras éthoxylés, les esters d'acides gras et de PEG, les glycérides partiels d'acides gras éthoxylés, les triglycérides d'acides gras polyglycérolés et leurs dérivés éthoxylés.

Les alcools gras éthoxylés sont de préférence choisis parmi les alcools gras en C18 à C22 et possédant de 6 à 12, et de préférence de 8 à 12 moles d'oxyde d'éthylène.

Comme alcools gras éthoxylés selon l'invention, on peut notamment citer par exemple les produits d'addition d'oxyde d'éthylène avec l'alcool béhénylique, notamment ceux comportant de 6 à 12 groupes oxyéthylénés (par exemple Beheneth-9 ou Beheneth-10 selon la dénomination du CTFA) ; les produits d'addition d'oxyde d'éthylène avec l'alcool stéarylique, notamment ceux comportant de 6 à 12 groupes oxyéthylénés (par exemple Steareth-9 selon la dénomination du CTFA) ; les produits d'addition d'oxyde d'éthylène avec l'alcool isostéarylique, notamment ceux comportant de 6 à 12 groupes oxyéthylénés (Isosteareth-9 selon la dénomination du CTFA) ; et leurs mélanges.

A titre de tensio-actifs non-ioniques oxyéthylénés et/ou glycérolés de HLB allant de 8 à 18, la composition peut également comprendre des alcools gras oxyéthylénés différents de ceux décrits ci-dessus à savoir, les produits d'addition d'oxyde d'éthylène avec l'alcool laurylique, notamment ceux comportant de 9 à 50 groupes oxyéthylénés (Laureth-9 à Laureth-50 en noms CTFA) ; les produits d'addition d'oxyde d'éthylène avec l'alcool cétéarylique ou cétylstéarylique (mélange d'alcool cétylique et d'alcool stéarylique), notamment ceux comportant de 9 à 30 groupes oxyéthylénés (Ceteareth-9 à Ceteareth-30 en noms CTFA) ; les produits d'addition d'oxyde d'éthylène avec l'alcool cétylique, notamment ceux comportant de 9 à 30 groupes oxyéthylénés (Ceteth-9 à Ceteth-30 en noms CTFA) ; et leurs mélanges. De préférence, l'alcool gras éthoxylé est l'alcool béhénique oxyéthyléné à 10 moles d'oxyde d'éthylène (Beheneth-10).

Les esters d'acide gras et de PEG sont de préférence choisis parmi les composés de formule (I) suivante :

R-COO-(CH₂-CH₂-O)ₘH (I)

où R est une chaîne hydrocarbonée linéaire ou ramifiée, saturée
ou insaturée, ayant de 10 à 24 atomes de carbone, et m est un nombre entier allant de 8 à 50.

Comme esters d'acides gras et de PEG, on peut citer par exemple les produits d'estérification avec les acides laurique, palmitique, stéarique ou béhénique, et leurs mélanges et avec l'oxyde d'éthylène, notamment ceux comportant de 9 à 50 groupes oxyéthylénés tels que les laurates de PEG-9 à PEG-50 (en noms CTFA : PEG-9 laurate à PEG-50 laurate) ; les palmitates de PEG-9 à PEG-50 (en noms CTFA : PEG-9 palmitate à PEG-50 palmitate) ; les stéarates de PEG-9 à PEG-50 (en noms CTFA : PEG-9 stéarate à PEG-50 stéarate) ; les palmito-stéarates de PEG-9 à PEG-50 ; les béhénates de PEG-9 à PEG-50 (en noms CTFA : PEG-9 behenate à PEG-50 behenate) ; et leurs mélanges.

L'émulsion directe (A1) peut aussi comprendre des tensioactifs additionnels. On peut à titre de tensioactifs additionnels utiliser les sels d'acides gras ayant 8 à 30 atomes de carbone, comme par exemple les sels d'acide palmitique, d'acide stéarique, d'acide béhénique ; les esters gras de glycérol, comme par exemple le glycéryl stéarate ; les dérivés oxyéthylénés des sels d'acides gras et des esters gras de glycérol, comportant 2 à 8 groupes oxyde d'éthylène, et leurs mélanges.

De préférence, le(s) tensioactif(s) non-ionique(s) oxyéthyléné(s) et/ou glycérolé(s) de HLB allant de 8 à 18 est (sont) choisi(s) parmi les alcools gras en C18-C22 possédant de 6 à 12, et de préférence 8 à 12 moles d'oxyde d'éthylène.

Encore plus préférentiellement, le système émulsionnant de l'émulsion (A1) ne contient pas d'autre(s) émulsionnant(s) que le(s) alcool(s) gras en C8-C22 possédant de 6 à 12 moles d'oxyde d'éthylène.

De manière générale, le (ou les) tensioactif(s) non-ionique(s) oxyéthyléné(s) et/ou glycérolé(s) de HLB allant de 8 à 18 sont présents en une quantité allant de 0,2 à 30 %, de préférence de 0,5 à 20 % et mieux de 1 à 10 % en poids par rapport au poids total de la composition finale.

Le rapport système émulsionnant/phase lipophile va de préférence de 0,04 à 0,8, encore plus préférentiellement de 0,1 à 0,7.

Comme indiqué plus haut, on entend par phase lipophile l'ensemble des constituants qui ne sont pas hydrophiles et qui sont différents des tensioactifs non ioniques oxyéthylénés et/ou glycérolés de HLB allant de 8 à 18.

La composition finale selon l'invention comprend de préférence une quantité de phase aqueuse allant de 30 à 95 % du poids total de la composition finale, cette quantité pouvant aller encore plus préférentiellement de 50 à 90 % en poids.

Cette phase aqueuse se partage entre la phase aqueuse de l'émulsion directe (A1) et la phase aqueuse contenant le ou les polymère(s) cationique(s).

De manière classique, la phase aqueuse peut contenir, outre l'eau, un ou plusieurs solvant(s) hydrosoluble(s) choisi(s) parmi les polyols (ou alcools polyhydriques), les alcools inférieurs hydrosolubles, et leurs mélanges.

On entend par "alcool inférieur", un alcool comportant de 1 à 8 atomes de carbone.

Comme polyols, on peut citer par exemple la glycérine ; les glycols comme le propylène glycol, le butylène glycol, l'isoprène glycol et les polyéthylène glycols tels que le PEG-8 ; le sorbitol ; les sucres tels que le glucose, le fructose, le maltose, le lactose, le sucrose ; et leurs mélanges.

Comme alcools inférieurs, on peut citer par exemple l'éthanol, l'isopropanol, le butanol et leurs mélanges.

Lorsqu'il(s) est (sont) présent(s) dans la composition de l'invention, le ou les solvant(s) peu(ven)t être en une quantité allant de 0,01 à 60 % en poids, de préférence de 0,5 à 50 % en poids et mieux de 5 à 20 % en poids par rapport au poids total de la phase aqueuse. Ces adjuvants ainsi que leurs concentrations doivent être tels qu'ils ne modifient pas la propriété recherchée pour la composition de l'invention.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire, qui soit font partie de la chaîne principale du polymère, soit sont portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques, réticulés ou non, et comportant au moins un des motifs de formules (I), (II), (III) ou (IV) suivantes : 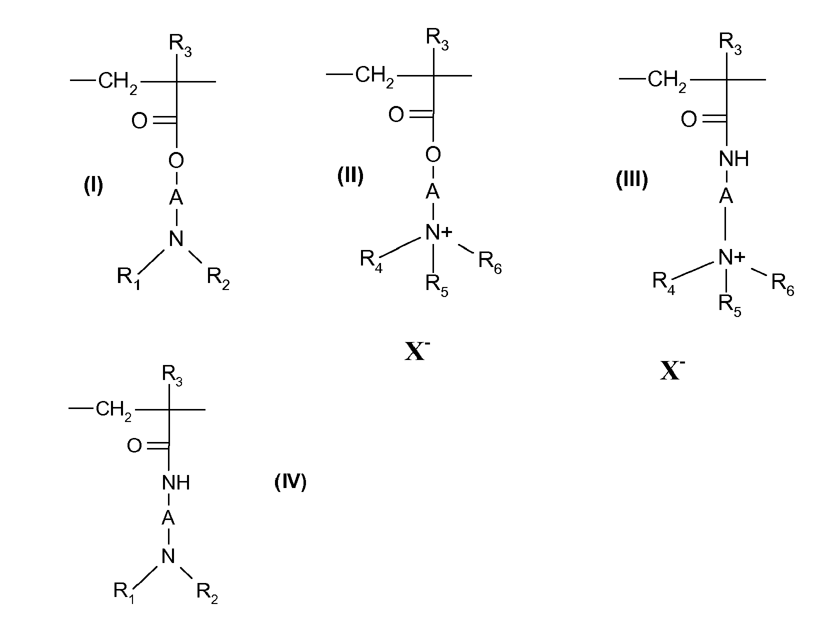 dans lesquelles :
   R₃ désigne un atome d'hydrogène ou un radical CH₃;
   A représente un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 6 atomes de carbone ou un radical benzyle et de préférence un groupe alkyle ayant de 1 à 6 atomes de carbone ;
   R₁ et R₂, identiques ou différents, représentent hydrogène ou un groupe alkyle ayant de 1 à 6 atomes de carbone et de préférence méthyle ou éthyle ;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motif(s) dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un halogénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle / vinylcaprolactame / vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - les copolymères vinylpyrrolidone / méthacrylamide de diméthylaminopropyle quaternisés tel que le produit vendu sous la dénomination "GAFQUAT HS 100" par la société ISP,
   - les polymères réticulés de sels de méthacryloyloxyalkyl(Cl-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide / chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société CIBA.
(2) Les polysaccharides cationiques notamment choisis parmi :
   a) les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
   b) les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylamidopropyl triméthylammonium, ou de diméthyl-diallylammonium.
      Les produits commercialisés répondant à cette définition sont plus particulièrement les produits vendus sous la dénomination "Celquat L 200" et "Celquat H 100" par la Société National Starch.
   c) les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(3) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(4) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(5) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-dialcoylamino hydroxyalcoyldialcoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique / diméthylaminohydroxypropyl / diéthylène triamine vendus sous la dénomination "Cartaretine F, F4 ou F8" par la société Sandoz.
(6) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 6 atomes de carbone. Le rapport molaire entre le polyalkylène polyamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique /époxypropyl / diéthylène-triamine.
(7) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 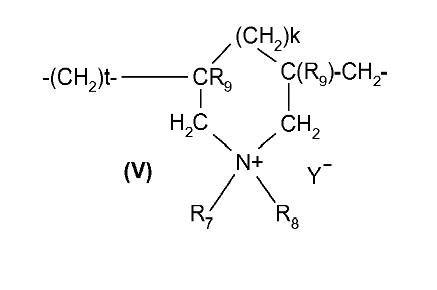 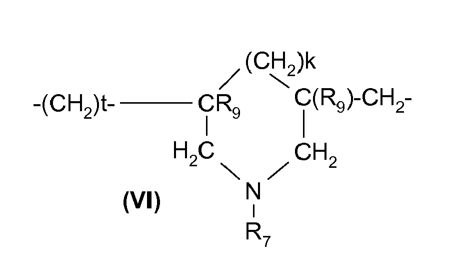 formules dans lesquelles
   k et t sont égaux à 0 ou 1, la somme k + t étant égale à 1 ;
   R₉ désigne un atome d'hydrogène ou un radical méthyle ;
   R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupements hétérocycliques, tels que pipéridinyle ou morpholinyle ; R₇ et R₈ indépendamment l'un de l'autre désignent de préférence un groupement alkyle ayant de 1 à 4 atomes de carbone ;
   Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallyl ammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(8) Le polymère de diammonium quaternaire contenant des motifs récurrents répondant à la formule (VII) : 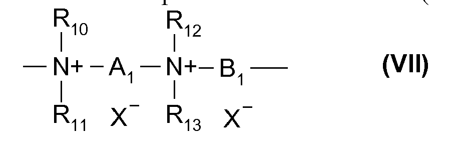 formule (VII) dans laquelle :
   R₁₀, R₁₁, R₁₂ et R₁₃, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆, linéaire ou ramifié, substitué par un groupement nitrile, ester, acyle, amide ou -CO-O-R₁₄-D ou -CO-NH-R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique ;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement

      -(CH₂)ₙ-CO-D-OC-(CH₂)n-

      dans lequel n est compris entre 1 et 100 et de préférence entre 1 et 50, et D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- .
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante : 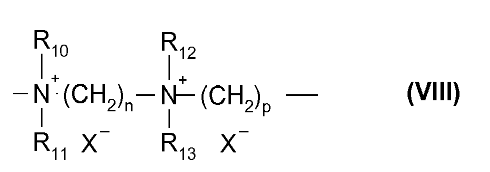 dans laquelle R₁₀, R₁₁, R₁₂ et R₁₃, identiques ou différents, désignent un radical alkyle ou hydroxyalkyle ayant de 1 à 4 atomes de carbone environ,
   n et p sont des nombres entiers variant de 2 à 20 environ et,
   X⁻ est un anion dérivé d'un acide minéral ou organique.
(9) Les polymères de polyammonium quaternaire constitués de motifs de formule (IX) : 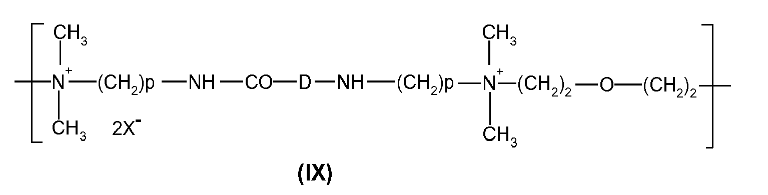 dans laquelle :
   p désigne un nombre entier variant de 1 à 6 environ,
   D peut être nul ou peut représenter un groupement -(CH₂)ᵣ -CO- dans lequel r désigne un nombre égal à 4 ou à 7, et
   X⁻ est un anion dérivé d'un acide minéral ou organique.
   Les polymères cationiques comportant des motifs de formule (IX) sont notamment décrits dans la demande de brevet EP-A-122 324 et peuvent être préparés selon les procédés décrits dans les brevets U.S.A. n° 4 157 388, 4 390 689, 4 702 906, 4 719 282.
   Parmi ces polymères, on préfère ceux de masse moléculaire mesurée par RMN du Carbone 13 inférieure à 100000, et dans la formule de laquelle :
   p est égal à 3, et,
      a) D représente un groupement -(CH₂)₄-CO- , X désigne un atome de chlore, la masse moléculaire mesurée par RMN du Carbone 13 ( RMN¹³C ) étant d'environ 5600 ; un polymère de ce type est proposé par la société MIRANOL sous le nom de MIRAPOL-AD1,
      b) D représente un groupement -(CH₂)₇-CO- , X désigne un atome de chlore, la masse moléculaire mesurée par RMN du Carbone 13 ( RMN¹³C ) étant d'environ 8100 ; un polymère de ce type est proposé par la société MIRANOL sous le nom de MIRAPOL-AZ1,
      c) D désigne la valeur zéro, X désigne un atome de chlore, la masse moléculaire mesurée par RMN du Carbone 13, ( RMN^{13C} ) étant d'environ 25500 ; un polymère de ce type est vendu par la société MIRANOL sous le nom MIRAPOL-A15,
      d) un " Block Copolymer " formé de motifs correspondant aux polymères décrits aux alinéas a) et c), proposé par la société MIRANOL sous les noms MIRAPOL-9, (masse moléculaire RMN¹³C, environ 7800) MIRAPOL-175, (masse moléculaire RMN¹³C, environ 8000) MIRAPOL-95, (masse moléculaire RMN¹³C, environ 12500).
   Plus particulièrement encore, on préfère selon l'invention le polymère à motifs de formule (XI) dans laquelle p est égal à 3, D désigne la valeur zéro, X désigne un atome de chlore, la masse moléculaire mesurée par RMN du Carbone 13, ( RMN^{13C} ) étant d'environ 25500.
(10) Les polymères quaternaires de vinylpyrrolidone et de vinylimidazole tels que par exemple les produits commercialisés sous les dénominations Luviquat FC 905, FC 550 et FC 370 par la société B.A.S.F.
(11) Les polyamines comme le Polyquart H vendu par HENKEL, référencé sous le nom de " POLYETHYLENEGLYCOL (15) TALLOW POLYAMINE " dans le dictionnaire CTFA.

D'autres polymères cationiques utilisables dans le cadre de l'invention sont des polyalkylèneimines, en particulier des polyéthylèneimines, des polymères contenant des motifs vinylpyridine
ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (7) et (8).

Le ou les polymère(s) cationique(s) selon l'invention se trouve(nt) en une quantité de 0,01 à 10 % en poids, de préférence de 0,1 à 5 % en poids, par rapport au poids total de la composition finale.

De préférence le rapport pondéral entre la quantité d'huiles végétale(s) et minérale(s) et la quantité de polymère(s) cationique(s) est supérieur à 1, de préférence compris entre 3 et 20.

### Additifs

La composition selon l'invention peut aussi contenir un ou plusieurs additif(s) cosmétique(s) couramment utilisé(s) dans la technique tels que, par exemple, les agents réducteurs, les agents oxydants, les corps gras différents de ceux définis ci-dessus, les silicones, les agents épaississants, les adoucissants, les agents anti-mousses, les agents hydratants, les agents émollients, les agents alcalinisants ou acidifiants, les plastifiants, les pigments, les charges minérales, les argiles, les minéraux colloïdaux, les nacres, les parfums, les peptisants, les conservateurs, les polymères fixants ou non, les polymères conditionneurs autres que les polymères cationiques définis ci-dessus, les protéines et les vitamines.

Les quantités de ces différents adjuvants sont celles classiquement utilisées dans le domaine considéré, et par exemple de 0,01 à 20 % du poids total de la composition finale.

Bien entendu, l'homme de l'art veillera à choisir le ou les éventuel(s) additif(s) de la composition selon l'invention de manière telle que les propriétés avantageuses attachées intrinsèquement à la composition conforme à l'invention ne soient pas ou substantiellement pas, altérées par l'addition envisagée.

La composition selon l'invention est de préférence une composition d'après shampoing à rincer ou non, ou un shampooing.

Lorsque la composition se trouve sous la forme d'un shampooing, la composition comporte de préférence un ou plusieurs tensioactif(s) anionique(s) et/ou amphotère(s).

Les tensioactifs anioniques pouvant être utilisés dans les compositions de l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

Les agents tensioactifs amphotères ou zwittérioniques, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (A) et (B) :

Rₐ-CONHCH₂CH₂-N(R_{b})(R_{c})(CH₂COO⁻) (A)

dans laquelle :
Rₐ représente un groupe alkyle dérivé d'un acide Rₐ-COOH présent dans l'huile de coprah hydrolysée, un groupe heptyle, nonyle ou undécyle,
   R_{b} représente un groupe bêta-hydroxyéthyle, et
   R_{c} représente un groupe carboxyméthyle ; et

   Rₐ'-CONHCH₂CH₂-N(B)(B') (B)

   dans laquelle :
   B représente -CH₂CH₂OX',
   B' représente -(CH₂)_{z}-Y', avec z = 1 ou 2,
   X' représente le groupe -CH₂CH₂-COOH ou un atome d'hydrogène,
   Y' représente -COOH ou le groupe -CH₂-CHOH-SO₃H,
   Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

La composition sous forme de shampooing peut aussi comprendre un ou plusieurs tensioactif(s) non-ionique(s) différent(s) des tensioactifs non-ioniques oxyéthylénés et/ou glycérolés de l'invention. En particulier, le(s) tensio-actif(s) peu(ven)t être choisi(s) parmi les alkylpolyglucosides.

Les compositions sous forme d'après-shampooing peuvent éventuellement comprendre un ou plusieurs agents tensioactifs cationiques de préférence non polymérique, non polymérique signifiant sans la répétition dans sa structure d'au moins une unité issue de la polymérisation d'au moins un monomère.

A titre d'exemples de tensioactif cationique, on peut notamment citer les sels d'amines grasses primaires, secondaires ou tertiaires, les sels d'ammonium quaternaire tels que les chlorures ou les bromures de tétraalkylammonium, d'alkylamidoalkyl trialkylammonium, de trialkylbenzylammonium, de trialkylhydroxy alkylammonium ou d'alkylpyridinium ; les dérivés d'imidazoline ; ou les oxydes d'amines à caractère cationique.

De préférence, les compositions selon l'invention ne contiennent pas de tensioactifs cationiques.

Les concentrations en tensioactifs autres que ceux de l'invention peuvent aller de 0,1 à 60 % en poids par rapport au poids de la composition finale, mieux de 0,2 à 40 % en poids. De préférence, lorsque la composition est un shampooing, cette concentration varie entre 4 et 30 % en poids par rapport au poids de la composition finale.

Le procédé de préparation des compositions de l'invention consiste à :
- préparer une émulsion directe (A1) comprenant le mélange d'huiles selon l'invention et un ou plusieurs tensioactif(s) non ionique(s) oxyéthyléné(s) et/ou glycérolé(s) de HLB allant de 8 à 18,
- ajouter cette émulsion à une composition aqueuse (A2) comprenant un ou plusieurs polymères cationiques.

La préparation de l'émulsion directe (A1) passe par une phase de chauffage au-dessus de la température d'inversion de phase du ou des tensioactifs de HLB allant de 8 à 18 puis par une phase de refroidissement en dessous de la température d'inversion de phase. Le mélange avec la phase aqueuse (A2) peut se faire à une température comprise entre la température ambiante et la température d'inversion de phase. Il se fait de préférence à température ambiante.

De préférence, le rapport pondéral (A1)/(A1 + A2) va de 0,1 à 0,9 et de préférence de 0,15 à 0,5.

De manière plus détaillée, on peut opérer de manière suivante :
1) Peser dans un récipient tous les constituants de l'émulsion (A1)
2) Homogénéiser le mélange, par exemple au moyen d'un Rayneri 350 tours/min, et chauffer en augmentant progressivement la température au moyen d'un bain marie jusqu'à une température supérieure à la température d'inversion de phase T1, c'est-à-dire jusqu'à l'obtention d'une phase transparente ou translucide (zone de microémulsion ou de phase lamellaire) puis d'une phase plus visqueuse qui indique l'obtention de l'émulsion inverse (E/H).
3) Arrêter le chauffage et maintenir l'agitation jusqu'à retour à la température ambiante, en passant par la température d'inversion de phase T1, c'est-à-dire la température à laquelle se forme une émulsion H/E fine.
4) Lorsque la température est redescendue en dessous de la zone d'inversion de Phase en Température (T1), additionner le ou les polymères cationiques et les éventuels additifs et en particulier les éventuels tensio-actifs additionnels, et les matières premières thermosensibles.

On obtient une composition finale stable dont les gouttelettes de phase lipophile sont fines avec des tailles de 10 à 200 nm, de préférence de 30 à 150 nm.

Dans la zone de formation d'une microémulsion (mélange translucide), les interactions hydrophiles et hydrophobes sont équilibrées car la tendance du tensioactif est de former aussi bien des micelles directes que des micelles inverses. Par chauffage au-delà de cette zone, il y a formation d'une émulsion E/H, car le tensioactif favorise la formation d'une émulsion eau-dans-huile. Puis lors du refroidissement en dessous de la zone d'inversion de phase, l'émulsion devient une émulsion H/E.

L'émulsification par inversion de phase est expliquée en détail dans l'ouvrage T. Fôrster, W. von Rybinski, A.Wadle, Influence of microemulsion phases on the preparation of fine disperse emulsions, Advances in Colloid and interface sciences, 58, 119-149, 1995 cité ici pour référence.

L'invention a encore pour objet un procédé de traitement cosmétique des matières kératiniques, telles que les cheveux consistant à appliquer sur celles-ci une composition telle que définie précédemment, puis à effectuer éventuellement un rinçage à l'eau.

La présente invention a également pour objet l'utilisation cosmétique de la composition telle que définie ci-dessus, pour le traitement cosmétique des matières kératiniques.

La présente invention a notamment pour objet l'utilisation cosmétique de la composition telle que définie ci-dessus, en tant qu'après-shampooing pour le soin des matières kératiniques.

La présente invention a enfin pour objet l'utilisation cosmétique de la composition telle que définie ci-dessus, en tant que shampooing pour le lavage des matières kératiniques.

Les exemples indiqués ci-dessous permettront de mieux comprendre l'invention sans toutefois présenter un caractère limitatif. Les quantités sont indiquées en % en poids sauf mention contraire. Dans ce qui suit, MA signifie Matière Active.

### EXEMPLES

### Mode opératoire pour réaliser les compositions de l'invention :

### 1) Préparation d'une émulsion d'huiles par le procédé PIT Emulsification

- Peser la phase lipophile et le(s) tensioactif(s) émulsionnant(s). Homogénéiser le mélange phase lipophile et tensioactif(s) émulsionnant(s) (A).
- Peser la phase aqueuse (B) séparément. Homogénéiser
- Chauffer le mélange phase lipophile et tensioactif(s) émulsionnant(s) (A) à T = 70 °C dans un bécher sous agitation magnétique.
- Chauffer la phase aqueuse (B) à T = 70 °C dans un bécher sous agitation magnétique.
- Verser (B) dans le mélange (A) sous agitation magnétique. L'émulsion se forme et le milieu devient blanc.

### Chauffage

- Augmenter progressivement le chauffage jusqu'à Tmax = 90 °C. On observe que le milieu devient translucide à T1<Tmax.

### Refroidissement

- Laisser refroidir sous agitation magnétique, en laissant revenir à température ambiante.

### 2) Préparation d'une formulation de shampooing

A température ambiante, mélanger le(s) tensio-actif(s) anionique(s) et/ou amphotère(s),

Ajouter la prédispersion de phase lipophile obtenue par le procédé PIT,

Ajouter le(s) polymère(s) cationique(s), l'eau résiduelle et éventuellement les autres additifs hydrosolubles.

### 3) Préparation d'une formulation de soin

A température ambiante, mélanger la prédispersion obtenue par le procédé PIT à de l'eau pour atteindre la concentration en phase lipophile voulue,

Ajouter le(s) polymère(s) cationique(s), l'eau résiduelle et éventuellement les autres additifs hydrosolubles.

**Exemple 1 : Shampooings**

| **Composition PIT 1** | |
|---|---|
| Alcool béhénique oxyéthyléné (10 OE) (EMULGIN BA 10, vendu par COGNIS) | 7 % MA |
| Huile d'olive (PURE OLIVE OIL, vendu par ARISTA INDUSTRIES) | 10 % MA |
| Huile de vaseline (MARCOL 82, vendu par EXXON MOBILE CHEMICAL) | 10 % MA |
| Eau | qsp100 % |

| **Formulation A (invention, avec polymère cationique)** | |
|---|---|
| Composition PIT 1 | 10 % |
| Lauryl éther sulfate (TEXAPON AOS 225 UP, vendu par COGNIS) | 4,9 %MA |
| Cocoyl amidopropyl bétaïne (TEGO BETAIN F 50, vendu par GOLDSCHMIDT) | 7,1 %MA |
| Chlorure de poly di-méthyl di-allyl ammonium à 40 % dans l'eau (MERQUAT 106, vendu par NALCO) | 0,4 %MA |
| Acide citrique | qsp pH 5 |
| Eau | qsp100 % |
| | |

| **Formulation B (comparative, sans polymère cationique)** | |
|---|---|
| Composition PIT 1 | 10 % |
| Lauryl éther sulfate (TEXAPON AOS 225 UP, vendu par COGNIS) | 4,9 % MA |
| Cocoyl amidopropyl bétaïne (TEGO BETAIN F 50, vendu par GOLDSCHMIDT) | 7,1 % MA |
| Acide citrique | qsp pH 5 |
| Eau | qsp100 % |

| | |
|---|---|
| 1 gramme de chaque composition est appliqué sur des mèches de 2,5 grammes de cheveux naturels. Une fois la composition appliquée, les mèches sont rincées et soumis à une première évaluation. | |

On observe sur cheveux humides que le toucher de la mèche traitée avec la composition A de l'invention est plus lisse par rapport à la mèche traitée avec la composition B comparative.

Les mèches sont ensuite séchées au casque pendant 15 minutes. On observe sur cheveux secs que le toucher de la mèche traitée avec la composition A de l'invention est plus lisse par rapport à la mèche traitée avec la composition B comparative.

**Exemple 2 : Shampooing**

| **Composition PIT 2** | |
|---|---|
| Alcool béhénique oxyéthyléné (10 OE) (EMULGIN BA 10, vendu par COGNIS) | 7 % MA |
| Huile de vaseline (MARCOL 82, vendu par EXXON MOBILE CHEMICAL) | 8 % MA |
| Huile d'olive (PURE OLIVE OIL, vendu par ARISTA INDUSTRIES) | 2 % MA |
| Eau | qsp100 % |
| | |

| **Formulation C (invention)** | |
|---|---|
| Composition PIT 2 | 10 % |
| Lauryl éther sulfate (TEXAPON AOS 225 UP, vendu par COGNIS) | 4,9 % MA |
| Cocoyl amidopropyl bétaïne (TEGO BETAIN F 50, vendu par GOLDSCHMIDT) | 7,1 % MA |
| Chlorure de poly di-méthyl di-allyl ammonium à 40 % dans l'eau (MERQUAT 106, vendu par NALCO) | 0,4 % MA |
| Acide citrique | qsp pH 5 |
| Eau | qsp100 % |

| | |
|---|---|
| 1 gramme de la composition est appliqué sur des mèches de 2,5 grammes de cheveux naturels. Une fois la composition appliquée, les mèches sont rincées puis séchées au casque pendant 15 minutes. On observe sur cheveux secs que la composition de l'invention apporte un bon lissage et un bon démêlage des cheveux. | |

**Exemple 3 : Soins capillaires**

| **Composition PIT 3** | |
|---|---|
| Alcool béhénique oxyéthyléné (10 OE) (EMULGIN BA 10, vendu par COGNIS) | 10 % MA |
| Huile d'olive (PURE OLIVE OIL, vendu par ARISTA INDUSTRIES) | 15 % MA |
| Huile de vaseline (MARCOL 82, vendu par EXXON MOBILE CHEMICAL) | 15 % MA |
| Eau | qsp100 % |
| | |

| **Formulation D (invention, avec polymère cationique)** | |
|---|---|
| Composition PIT 3 | 50 % |
| Chlorure de poly di-méthyl di-allyl ammonium à 40 % dans l'eau (MERQUAT 106, vendu par NALCO) | 1 % MA |
| Acide citrique | qsp pH 5 |
| Eau | qsp100 % |

| **Formulation E (comparative, sans polymère cationique)** | |
|---|---|
| Composition PIT 3 | 50 % |
| Acide citrique | qsp pH 5 |
| Eau | qsp100 % |

| | |
|---|---|
| 1 gramme de chaque composition est appliqué sur des mèches de 2,5 grammes de cheveux naturels. Une fois la composition appliquée, les mèches sont rincées et soumis à une première évaluation. | |

On observe sur cheveux humides que le toucher de la mèche traitée avec la composition D de l'invention est plus lisse par rapport à la mèche traitée avec la composition E comparative.

Les mèches sont ensuite séchées au casque pendant 15 minutes. On observe sur cheveux secs que le toucher de la mèche traitée avec la composition D de l'invention est plus lisse par rapport à la mèche traitée avec la composition E comparative.

**Exemple 4 : Soin capillaire**

| **Formulation F (invention, avec polymère cationique)** | |
|---|---|
| Composition PIT 3 | 50 % |
| Homopolymère de chlorure de méthacrylate d'éthyl tri-méthyl ammonium réticulé, en dispersion dans un mélange d'esters à 50 % (SALCARE SC 96, vendu par CIBA) | 1,25 %MA |
| Acide citrique | qsp pH 5 |
| Eau | qsp100 % |

| | |
|---|---|
| 1 gramme de chaque composition est appliqué sur des mèches de 2,5 grammes de cheveux naturels. Une fois la composition appliquée, les mèches sont rincées et soumis à une première évaluation. On observe sur cheveux humides que le toucher de la mèche traitée avec la composition F de l'invention est plus lisse par rapport à la mèche traitée avec la composition E comparative. | |

Les mèches sont ensuite séchées au casque pendant 15 minutes. On observe sur cheveux secs que le toucher de la mèche traitée avec la composition F de l'invention est plus lisse par rapport à la mèche traitée avec la composition E comparative.

## Revendications

1. Composition cosmétique aqueuse pour le traitement des matières kératiniques, telles que les cheveux, comprenant une émulsion directe (A1) obtenue par un procédé PIT et un ou plusieurs polymères cationiques, **caractérisée par le fait que** l'émulsion directe (A1) comporte :
a) une phase lipophile comprenant une ou plusieurs huile(s) minérale(s) et une ou plusieurs huile(s) végétale(s),
b) un système émulsionnant comprenant un ou plusieurs tensioactif(s) non ionique(s) oxyéthyléné(s) et/ou glycérolé(s) de HLB allant de 8 à 18, et
c) une phase aqueuse.

2. Composition cosmétique selon la revendication 1, **caractérisée en ce que** le rapport pondéral de la quantité d'huiles minérale(s) et végétale(s) sur la quantité de tensioactif(s) non ionique(s) oxyéthyléné(s) est inférieur à 4.

3. Composition cosmétique selon la revendication 1 ou 2, **caractérisée en ce que** l'huile végétale est choisie parmi :
- l'huile d'amande douce,
- l'huile d'argan,
- l'huile d'avocat,
- l'huile d'arachide,
- l'huile de camélia,
- l'huile de carthame,
- l'huile de calophyllum,
- l'huile de colza,
- l'huile de coprah,
- l'huile de coriandre,
- l'huile de courge,
- l'huile de germes de blé,
- l'huile de jojoba,
- l'huile de lin,
- l'huile de macadamia,
- l'huile de germes de maïs,
- l'huile de noisette,
- l'huile de noix,
- l'huile de vernonia,
- l'huile de noyau d'abricot,
- l'huile d'olive,
- l'huile d'onagre,
- l'huile de palme,
- l'huile de passiflore,
- l'huile de pépins de raisin,
- l'huile de rosier,
- l'huile de ricin,
- l'huile de seigle,
- l'huile de sésame,
- l'huile de son de riz,
- l'huile de soja, et
- l'huile de tournesol.

4. Composition cosmétique selon l'une quelconque des revendications précédentes, **caractérisée en ce que** l'huile minérale est choisie parmi :
- les mélanges d'huiles hydrocarbonées dérivées du pétrole
- l'huile de paraffine, volatile ou non volatile,
- l'huile de vaseline,
- les polyoléfines et en particulier les polydécènes,
- les isoparaffines telles que l'isohexadécane, l'isododécane et les polyisobutylènes hydrogénés.

5. Composition cosmétique selon l'une quelconque des revendications précédentes, **caractérisée en ce que** l'huile minérale est l'huile de vaseline et l'huile végétale est choisie parmi l'huile d'olive, l'huile d'argan, l'huile d'avocat, l'huile de colza, l'huile de jojoba, l'huile de soja, l'huile de tournesol.

6. Composition cosmétique selon l'une quelconque des revendications précédentes, **caractérisée en ce que** l'huile (les huiles) végétale(s) et l'huile (les huiles) minérale(s) sont présentes dans la composition dans un rapport pondéral huile(s) végétale(s)/huile(s) minérale(s) inférieur ou égal à 1.

7. Composition cosmétique selon l'une quelconque des revendications précédentes, **caractérisée en ce que** la concentration en huiles minérale(s) et végétale(s) va de 0,1 à 30 % en poids, du poids total de la composition finale.

8. Composition cosmétique selon l'une quelconque des revendications précédentes, **caractérisée en ce que** le(s) tensioactif(s) non ionique(s) oxyéthyléné(s) et/ou glycérolé(s) de HLB allant de 8 à 18 est (sont) choisi(s) parmi les alcools gras en C18 à C22 et possédant de 6 à 12, et de préférence de 8 à 12 moles d'oxyde d'éthylène.

9. Composition cosmétique selon l'une quelconque des revendications précédentes, **caractérisée en ce que** le tensioactif non ionique oxyéthyléné et/ou glycérolé de HLB allant de 8 à 18 est l'alcool béhénique oxyéthyléné à 10 moles d'oxyde d'éthylène.

10. Composition cosmétique selon l'une quelconque des revendications précédentes, **caractérisée en ce que** la concentration en tensioactif(s) non ionique(s) oxyéthyléné(s) et/ou glycérolé(s) de HLB allant de 8 à 18 va de 0,2 à 30 % en poids, du poids total de la composition.

11. Composition selon l'une quelconque des revendications précédentes, **caractérisée par le fait que** le polymère cationique est choisi parmi :
(1) Les homopolymères ou copolymères dérivés d'esters ou d'amides acryliques ou méthacryliques, réticulés ou non, et comportant au moins un des motifs de formules (I), (II), (III) ou (IV) suivantes : 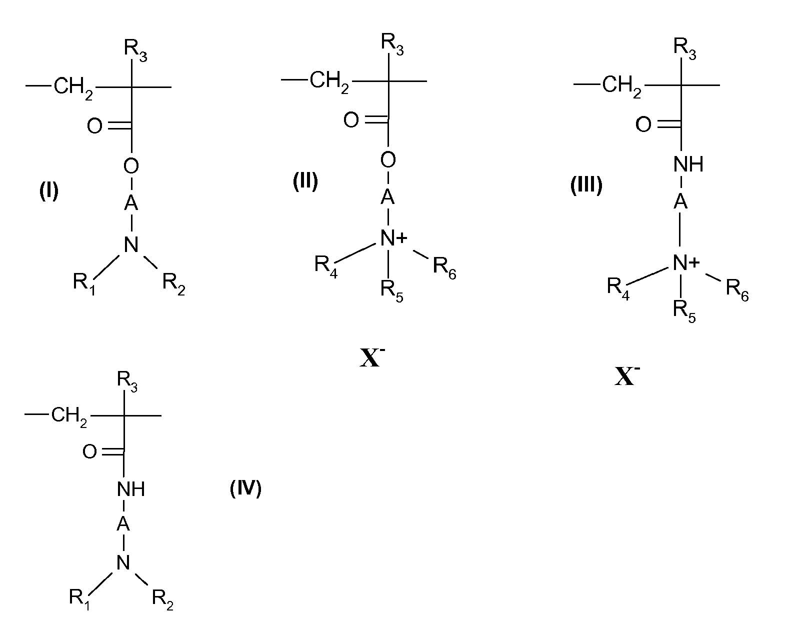 dans lesquelles :
R3 désigne un atome d'hydrogène ou un radical CH3 ;
A représente un groupe alkyle, linéaire ou ramifié, de 1 à 6 atomes de carbone, de préférence 2 ou 3 atomes de carbone ou un groupe hydroxyalkyle de 1 à 4 atomes de carbone ;
R4, R5, R6, identiques ou différents, représentent un groupe alkyle ayant de 1 à 6 atomes de carbone ou un radical benzyle et de préférence un groupe alkyle ayant de 1 à 6 atomes de carbone ;
R1 et R2, identiques ou différents, représentent hydrogène ou un groupe alkyle ayant de 1 à 6 atomes de carbone et de préférence méthyle ou éthyle ;
X désigne un anion dérivé d'un acide minéral ou organique tel qu'un anion méthosulfate ou un halogénure tel que chlorure ou bromure ;
(2) Les polysaccharides cationiques ;
(3) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères ;
(4) Les polyaminoamides solubles dans l'eau préparés en particulier par polycondensation d'un composé acide avec une polyamine ;
(5) Les dérivés de polyaminoamides résultant de la condensation de polyalcoylènes polyamines avec des acides polycarboxyliques suivie d'une alcoylation par des agents bifonctionnels ;
(6) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 6 atomes de carbone ;
(7) Les cyclopolymères d'alkyl diallyl amine ou de dialkyl diallyl ammonium ;
(8) Le polymère de diammonium quaternaire contenant des motifs récurrents répondant à la formule : 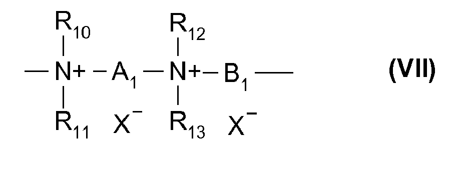 formule (VII) dans laquelle :
R₁₀, R₁₁, R₁₂ et R₁₃, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O-R₁₄-D ou -CO-NH-R₁₄-D où R₁₄ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement
-(CH₂)ₙ-CO-D-OC-(CH₂)ₙ-
dans lequel n est compris entre 1 et 100 et de préférence entre 1 et 50, et D désigne :
a) un reste de glycol de formule : -O-Z-O-, où Z désigne un radical hydrocarboné linéaire ou ramifié ou un groupement répondant à l'une des formules suivantes :
-(CH₂-CH₂-O)ₓ-CH₂-CH₂-
-[CH₂-CH(CH₃)-O]_{y}-CH₂-CH(CH₃)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-,
où Y désigne un radical hydrocarboné linéaire ou ramifié, ou bien le radical bivalent
- CH₂-CH₂-S-S-CH₂-CH₂- ;
d) un groupement uréylène de formule : -NH-CO-NH- ;
(9) Les polymères de polyammonium quaternaire constitués de motifs de formule (IX) : 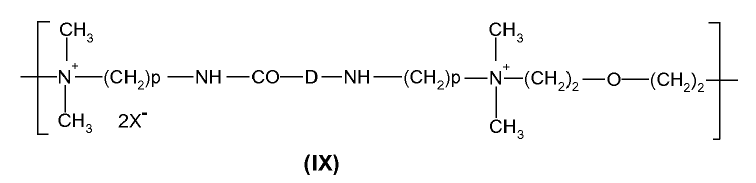 dans laquelle :
p désigne un nombre entier variant de 1 à 6 environ,
D peut être nul ou peut représenter un groupement -(CH₂)ᵣ -CO- dans lequel r désigne un nombre égal à 4 ou à 7, et
X⁻ est un anion dérivé d'un acide minéral ou organique ;
(10) Les polymères quaternaires de vinylpyrrolidone et de vinylimidazole ;
(11) Les polyamines.

12. Composition selon l'une quelconque des revendications précédentes, **caractérisée par le fait que** les polymères cationiques se trouvent en une quantité de 0,01 à 10 % en poids par rapport au poids total de la composition finale.

13. Procédé de préparation d'une composition telle que définie à l'une quelconque des revendications 1 à 12, consistant à :
- préparer une émulsion directe (A1) comprenant le mélange d'huiles selon l'invention et un ou plusieurs tensioactif(s) non ionique(s) oxyéthyléné(s) et/ou glycérolé(s) de HLB allant de 8 à 18,
- ajouter cette émulsion à une composition aqueuse (A2) comprenant un ou plusieurs polymères cationiques.

14. Procédé de traitement des matières kératiniques, telles que les cheveux, **caractérisé par le fait que** l'on applique sur celles-ci une composition telle que définie à l'une quelconque des revendications 1 à 12, puis que l'on effectue éventuellement un rinçage à l'eau.

15. Utilisation cosmétique d'une composition cosmétique telle que définie à l'une quelconque des revendications 1 à 12, en tant qu'après-shampooing pour le soin des matières kératiniques, ou en tant que shampooing pour le lavage des matières kératiniques.
